(19) Europäisches Patentamt
European Patent Office
Office européen des brevets

(11) Publication number: 0 307 222 A2

# (12) EUROPEAN PATENT APPLICATION

(21) Application number: 88308346.1

(22) Date of filing: 09.09.88

(51) Int. Cl.4: C 07 D 493/22 //(C07D493/22,313:00,311:00, 311:00,307:00)

(30) Priority: 11.09.87 GB 8721374

(43) Date of publication of application: 15.03.89 Bulletin 89/11

(84) Designated Contracting States: AT BE CH DE ES FR GB GR IT LI LU NL SE

(71) Applicant: AMERICAN CYANAMID COMPANY
One Cyanamid Plaza
Wayne New Jersey 07470 (US)

(72) Inventor: Freeman, Stephen
2 Coronation Cottages Downley Common Downley
High Wycombe Buckinghamshire (GB)

(74) Representative: Skailes, Humphrey John et al
Frank B. Dehn & Co. Imperial House 15-19 Kingsway
London WC2B 6UZ (GB)

## (54) Process for the preparation of macrolide compounds.

(57) A process for preparing a compound of formula (I)

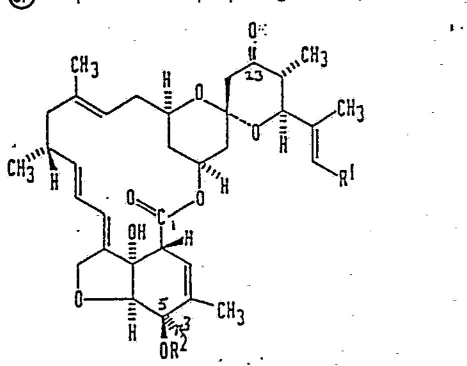

(I)

(in which $R^1$ represents methyl, ethyl or isopropyl group, $R^2$ represents a hydrogen atom or a methyl or acetyl group and $R^3$ represents a hydrogen atom, or $OR^2$ and $R^3$ together with the carbon atom to which they are attached represent the group >C=0) which comprises oxidising a corresponding 23α-OH compound in the presence of a phase transfer catalyst.

EP 0 307 222 A2

## Description

### Process for the preparation of macrolide compounds

This invention relates to a novel process for the preparation of antibiotic compounds.

The compounds of formula (I)

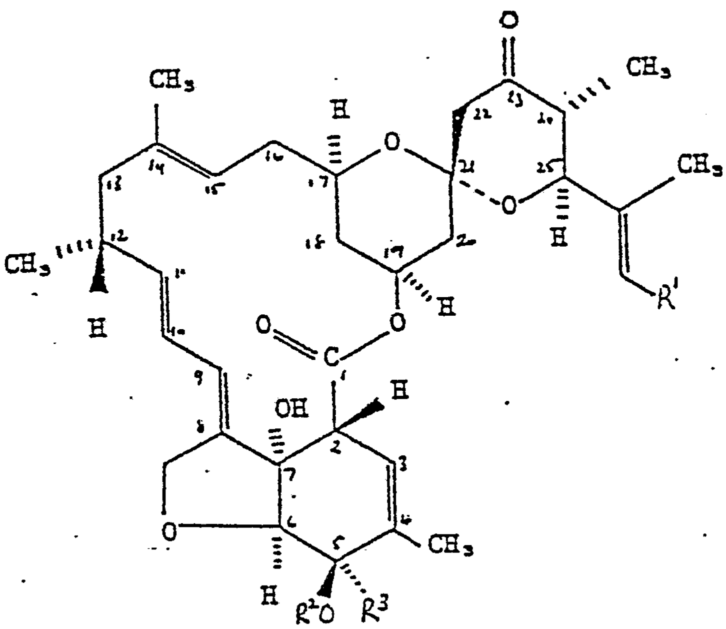

(I)

in which $R^1$ represents methyl, ethyl or isopropyl group, $R^2$ represents a hydrogen atom or a methyl or acetyl group and $R^3$ represents a hydrogen atom are described in UK Patent Specification 2176182A, and the corresponding 5-keto compounds in which $OR^2$ and $R^3$ together with the carbon atom to which they are attached represent the group $>C=0$ are described in EP-A 0238258.

These compounds have antibiotic, particularly anti-endoparasitic, anti-ectoparasitic, anti-fungal, insecticidal, nematicidal and acaricidal activity and are useful in combating parasites in animals and humans and pests in agriculture, horticulture, forestry, public health and stored products. The compounds may also be used as intermediates in the preparation of other active compounds.

The present invention provides a novel and useful synthesis of the compounds of formula (I) from fermented starting materials. The process is convenient to use and provides the compounds of formula (I) in good yield.

Thus, we provide a process for preparing a compound of formula (I) which comprises oxidising a compound of formula (II)

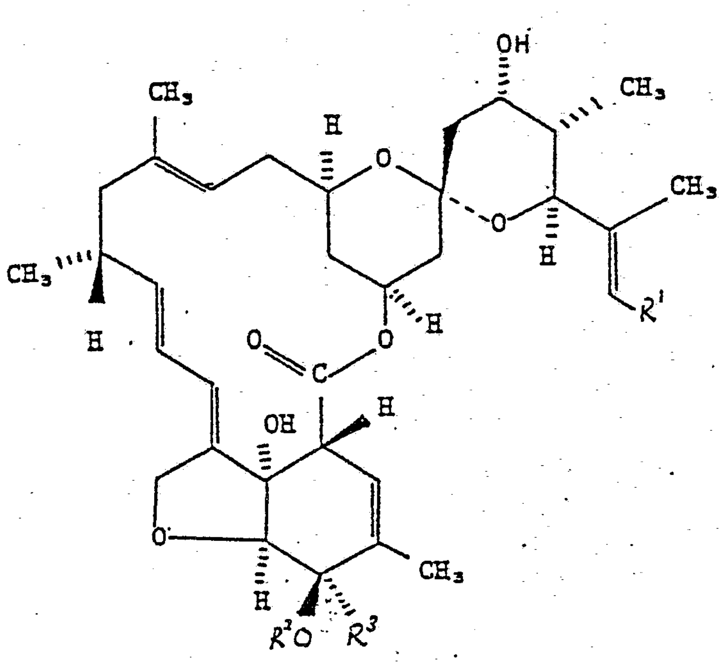

(II)

(wherein $R^1$, $R^2$ and $R^3$ are as defined previously) in the presence of a phase transfer catalyst, followed by deacetylation of a compound of formula (I) in which $OR^2$ is an acetyloxy group when a compound of formula (I) in which $OR^2$ is a hydroxy group is required.

The reaction may be effected using an oxidising agent such as a chromium (VI) oxidising agent, e.g. sodium or pyridinium dichromate.

Examples of suitable phase transfer catalysts which may conveniently be employed with such oxidising agents include tetraalkylammonium salts such as tetra-n-butylammonium salts e.g. tetra-n-butylammonium hydrogen sulphate.

The reaction may conveniently be effected in a suitable solvent, for example an ester such as ethyl acetate and is preferably carried out in the presence of a strong acid such as a mineral acid e.g. sulphuric acid.

The reaction may be carried out at a temperature in the range of from -80°C to +50°C, e.g. 0°C.

Deacetylation to provide a compound of formula (I) in which $OR^2$ is a hydroxy group may be performed using base hydrolysis e.g. using sodium or potassium hydroxide in aqueous alcohol or by acid hydrolysis e.g. using concentrated sulphuric acid in methanol.

The compounds of formula (II) in which $R^1$ is as defined previously and $OR^2$ represents a hydroxy or methoxy group may be obtained using the fermentation and isolation methods described in UK Patent Specification No. 2166436A. Compounds of formula (II) in which $OR^2$ represents an acetyloxy group may be prepared from the corresponding 5-OH compounds using standard acetylation procedures.

Thus, for example, acetylation may be effected using an acetylating agent such as acetic acid or a reactive derivative thereof, such as an acetyl halide (e.g. acetyl chloride), anhydride or activated ester, or a reactive derivative of a carbonic acid $CH_3OCOOH$ or thiocarbonic acid $CH_3OCSOH$.

Acetylations employing acetyl halides and anhydrides may if desired be effected in the presence of an acid binding agent such as a tertiary amine (e.g. triethylamine, dimethylaniline or pyridine), inorganic bases (e.g. calcium carbonate or sodium bicarbonate), and oxiranes such as lower 1,2-alkylene oxides (e.g. ethylene oxide or propylene oxide) which bind hydrogen halide liberated in the acetylation reaction.

Acetylation employing acetic acid is desirably conducted in the presence of a condensing agent, for example a carbodiimide such as N,N′-dicyelohexylcarbodiimide or N-ethyl-N′γ-dimethylaminopropylcarbodiimide; a carbonyl compound such as carbonyldiimidazole; or an isoxazolium salt such as N-ethyl-5-phenylisoxazolium perchlorate.

An activated ester may conveniently be formed in situ using, for example, 1-hydroxybenzotriazole in the presence of a condensing agent as set out above. Alternatively, the activated ester may be preformed.

The acetylation reaction may be effected in aqueous or non-aqueous reaction media, conveniently at a temperature in the range -20° to +100°C, e.g. -10° to +50°C.

The compounds of formula (II) in which $OR^2$ and $R^3$ together with the carbon atom to which they are attached represent >C=0 (hereinafter referred to as '5-keto compounds of formula (II)′) may be prepared by cultivating Streptomyces thermoarchaensis NCIB 12015 or a mutant thereof and isolating the compound from the fermentation broth so obtained.

The Streptomyces organism may be cultured by conventional means, i.e. in the presence of assimilable sources of carbon, nitrogen and mineral salts. Assimilable sources of carbon, nitrogen and minerals may be

provided by either simple or complex nutrients for example as described in UK Patent Specification 2166436A.

Cultivation of the Streptomyces organism will generally be effected at a temperature of from 20 to 50°C preferably from 25 to 40°C, and will desirably take place with aeration and agitation e.g. by shaking or stirring. The medium may initially be inoculated with a small quantity of a sporulated suspension of the microorgansim but in order to avoid a growth lag a vegetative inoculum of the organism may be prepared by inoculating a small quantity of the culture medium with the spore form of the organism, and the vegetative inoculum obtained may be transferred to the fermentation medium, or, more preferably to one or more seed stages where further growth takes place before transfer to the principal fermentation medium. The fermentation will generally be carried out in the pH range 5.5 to 8.5.

The fermentation may be carried out for a period of 2-10 days, e.g. about 5 days.

The 5-keto compounds of formula (II) may be separated from the whole fermentation broth so obtained by conventional isolation and separation techniques. A variety of fractionation techniques may be used, for example adsorption-elution, precipitation, fractional crystallisation, solvent extraction and liquid-liquid partition which may be combined in various ways. Solvent extraction, partition between two solvents which are immiscible or only partially miscible with each other and chromatography have been found to be most suitable for isolating and separating the compound. A suitable method for obtaining the 5-keto compounds of formula (II) using these procedures is described in British Patent Specification 2187742A.

The invention is illustrated but not limited by the following Example in which temperatures are in °C.

'Factor A' is the compound of formula (II) in which $R^1$ represents isopropyl and $OR^2$ is a hydroxy group. The preparation of 5-keto Factor A is described in British Patent Specification 2187742A.

Example 1

5,23-Diketo Factor A

An ice-cold solution prepared from concentrated sulphuric acid (1.2ml) and sodium dichromate (120mg) in water (2ml) was added over 15 min to an ice-cold solution of 5-keto Factor A (200mg) and tetrabutylammonium hydrogen sulphate (15mg) in ethyl acetate (4ml) with vigorous stirring. After 1h the mixture was diluted with ethyl acetate and the organic phase was washed with saturated aqueous sodium bicarbonate. The dried organic phase was evaporated and the gum was purified by chromatography over Merck Keiselgel 60 230-400 mesh (100ml). Elution with 10% ethyl acetate in dichloromethane afforded the title compound as a pale yellow foam (86mg) δ ($CDCl_3$) includes 6.57 (m,1H); 2.50 (s,2H); and 1.89 (m,3H).

**Claims**

1. A process for preparing a compound of formula (I)

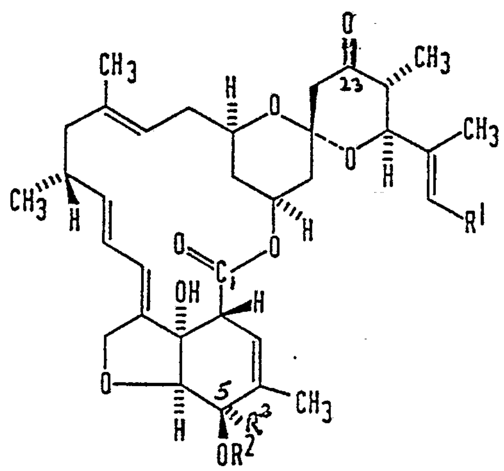

(I)

(in which $R^1$ represents methyl, ethyl or isopropyl group, $R^2$ represents a hydrogen atom or a methyl or acetyl group and $R^3$ represents a hydrogen atom, or $OR^2$ and $R^3$ together with the carbon atom to which they are attached represent the group >C=O) which comprises oxidising a compound of formula (II)

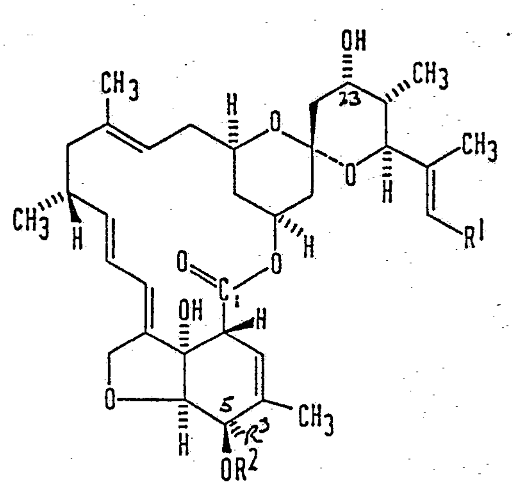

(2)

(wherein $R^1$, $R^2$ and $R^3$ are as defined previously) in the presence of a phase transfer catalyst, followed by deacetylation of a compound of formula (I) in which $OR^2$ is an acetyloxy group when a compound of formula (I) in which $OR^2$ is a hydroxy group is required.

2. A process according to claim 1 in which the phase transfer catalyst is a tetra-alkylammonium salt.

3. A process according to claim 1 in which the phase transfer catalyst is a tetra-n-butylammonium salt.

4. A process according to claim 1 in which sodium or pyridinium dichromate is used as the oxidising agent.

5. A process according to claim 1 in which the reaction is carried out in the presence of a mineral acid.

6. A process according to claim 1 in which the reaction is carried out in an ester solvent.